(19) Europäisches Patentamt
European Patent Office
Office européen des brevets

(11) **EP 2 600 919 B1**

(12) **EUROPEAN PATENT SPECIFICATION**

(45) Date of publication and mention
of the grant of the patent:
**14.09.2016   Bulletin 2016/37**

(51) Int Cl.:
***A61M 1/36*** *(2006.01)*     ***B01D 39/00*** *(2006.01)*

(21) Application number: **11755119.2**

(86) International application number:
**PCT/IB2011/001792**

(22) Date of filing: **29.07.2011**

(87) International publication number:
**WO 2012/017291 (09.02.2012 Gazette 2012/06)**

(54) **IMPROVED FILTER FOR REMOVING SUBSTANCES FROM BLOOD OR FROM BLOOD DERIVATIVES, AND A METHOD FOR OBTAINING IT**

VERBESSERTES FILTER ZUR ENTFERNUNG VON STOFFEN AUS BLUT ODER BLUTDERIVATEN SOWIE VERFAHREN ZU SEINER HERSTELLUNG

FILTRE AMÉLIORÉ POUR EXTRAIRE DES SUBSTANCES DU SANG OU DE DÉRIVÉS DU SANG, ET PROCÉDÉ DE FABRICATION ASSOCIÉ

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority:  **06.08.2010   IT MI20101514**

(43) Date of publication of application:
**12.06.2013   Bulletin 2013/24**

(73) Proprietor: **GVS S.p.A.**
**40069 Zola Predosa (Bologna) (IT)**

(72) Inventors:
• **SCAGLIARINI, Massimo**
**40033 Casalecchio Di Reno (Bologna) (IT)**

• **QUERZÉ, Luca**
**40068 S. Lazzaro Di Savena (Bologna) (IT)**

(74) Representative: **Ripamonti, Enrico**
**Giambrocono & C. S.p.A.,**
**Via Rosolino Pilo, 19/B**
**20129 Milano (IT)**

(56) References cited:
**EP-A1- 1 666 129        EP-A1- 1 897 571**
**WO-A1-2005/113136    CN-C- 100 346 861**
**JP-A- 1 224 324          US-A1- 2002 122 939**

EP 2 600 919 B1

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

The present invention relates to a filter for removing substances, in particular such as leukocytes and platelets, from blood or from blood derivatives, in accordance with the introduction to the main claim. The invention also relates to a method for forming the aforesaid filter, in accordance with the corresponding independent claim.

Filtration of blood components for transfusion is a known practice usually performed on the blood withdrawn from a donor. The main purpose of this filtration or purification is to reduce as much as possible the presence of leukocytes in the transfused blood (leukodepletion) and the presence of platelets therein in order to reduce possible deleterious effects related to transfusion, such as: alloimmunization; non-hemolytic febrile transfusion reaction prevention; infection by cytomegalovirus; cytokine release reduction and others.

It has therefore become common, and indeed is a practice recommended and prescribed by regulatory bodies, to use filters for leukodepletion in preparing blood components.

With particular reference to these products, currently available filters comprise a casing, generally of plastic material, presenting an inlet port and an outlet port both connected to tubes through which the blood entering and leaving the casing flows. This casing contains a usually layered filter element consisting generally of mesh, membranes or more typically non-woven fabrics. The polymers most commonly used for the non-woven fabrics are polyesters, specifically PBT (polybutyleneterephthalate). US4701267 describes a filter unit for removing leukocytes from a suspension containing them, and in particular describes a filter element using a non-woven fabric with fibres of particular dimensions and density. This patent describes the materials specifically used for producing the constituent fibres of the non-woven fabric.

EP329303 describes a method and device for leukodepletion of a platelet concentrate deriving from blood; the text describes various filter means obtained in PTFE (polytetrafluoroethylene), in PBT or in other fibres with hydroxyl or anionic groups. In particular, the use of polyester, polyamide and acrylic fibres is described.

The filter material is usually obtained by aggregating filtering layers which differ by physical characteristics, such as air permeability, average pore dimension, average fibre dimension and surface tension. Filtration results in leukocyte removal both by physico-mechanical action (screening) related to the cell dimensions (screen filtration), by the capacity of the leukocytes to adhere to the synthetic fibres (depth filtration) independently of their dimensions, and by the effect of biological phenomena related to the presence of protein molecules having adhesive properties present on the external surface of their cell membranes, (intergrine, selecting, immunoglobulin-like). The surface tension of the filter material is of extreme importance in determining the hydraulic strength of the filter and in promoting or not promoting the adsorption mechanism which governs the effectiveness of removing the particular substance (leukocytes) from the blood.

In this respect, it is known that high material surface tension reduces the platelet adhesion phenomenon during blood filtration. In contrast, low surface tension of the filter material gives rise to a hydrophobic interaction with the filtered liquid, with a greater pressure drop across the filter (less throughput for equal pressure difference) and a lack of permeation uniformity, with relative formation of channels via preferential circuits.

Filters of the aforestated types and methods for their production are known. For example, EP1897571 describes a filter for removing substances from blood or blood derivatives comprising a filtering polymer material having a polyurethane coating, wherein this latter has an average molecular weight not exceeding 10,000 Dalton (Da). This polyurethane is obtained by an addition reaction of an aliphatic diisocyanate, a polyethylene glycol and butanediol, using an excess of this latter, so that the addition reaction is stoichiometrically unbalanced.

This known filter is obtained by a method involving the use of pollutant chemical products requiring particular attention for their disposal. This results in an increase in production costs and hence in the cost of the final product obtained.

Another patent text, WO2005/113136, describes a polyurethane material for leukocyte adsorption having an average molecular weight equal to or exceeding 20,000 Da; this prior text states that the use of a polyurethane of molecular weight less than 20,000 Da is not adequate as this molecular weight is too low for a material which is to be used for leukodepletion.

Both these prior patents hence describe leukodepletion filters using filter elements having at least one layer coated with polyurethane. However this coating can present either a molecular weight which may be insufficient to adequately filter the blood, or on the contrary a molecular weight which is adequate, according to that stated by the inventors, but the application of which to the non-woven fabric uses methods involving the use of chemical solvents with consequent high risks to the environment. Hence for their implementation, these methods require means to prevent release of pollutant products into the atmosphere, these means representing a certain cost which is transferred to the finished product.

An object of the present invention is to provide a filter able to remove substances from the blood, and a method for its formation, which represent an improvement over similar already known filters and methods.

A particular object of the invention is to provide a filter having high leukocyte retention and high platelet retention, with extremely low undesired release and optimal filtration times, and obtainable by a method which respects the environment and is of low environmental impact.

Another object is to provide a method of the stated type which is of low implementation costs and high efficiency.

These and other objects which will be apparent to the expert of the art are attained by a filter in accordance with the accompanying claims.

According to the invention, the filter is made of non-woven fabric consisting of a layer (or hydrophobic substrate) of PBT having low surface tension and high hydrophobicity. This filter comprises at least one layer of this material.

Said layer is coated with polyurethane material of which the molecular weight is between 12,000 Da and 18,000 Da and preferably between 15,000 Da and 17,500 Da. In this respect it has been surprisingly found that with Polyurethane of molecular weight within the aforesaid ranges, a filter can be formed for removing leukocytes and platelets from the blood. In particular, with these values it has been shown possible to reduce the total number of leukocytes by a value of the order of $10^4$ - $10^6$ times.

The polyurethane coating is applied to the hydrophobic substrate by an impregnation technique, by immersing this latter in a container in which a mixture is present containing the polyurethane in a controlled concentration. The mixture consists of a solution in which the polyurethane is dissolved in an inert polar organic solvent, such as isopropanol or propylene glycol methyl ether, or preferably a dispersion in water. The polyurethane concentration in said mixture is from 0.5% to 20% by weight, preferably around 8%, whether in organic solution or in aqueous dispersion.

Alternatively, solutions or suspensions in mixtures of inert polar organic solvent and water can be used.

The following are used as polyurethane components: an aliphatic isocyanate (isocyanate -N=C=O group) with characteristics similar to diphenylmethane diisocyanate (MDI); a polyol (typically a polyethylene glycol or polyesters) with which the isocyanate forms a urethane bond.

The impregnation technique is conventional and consists of immersing the non-woven fabric in a tank containing the mixture. The effect of the polyurethane coating on the non-woven fabric layer is that the surface tension of this latter increases. The layer hence loses the hydrophobicity characteristic and becomes hydrophilic and easily wettable with water. In addition the coating of the non-woven fabric enables the average pore diameter of the filtering layer to be controlled such that it is between 3 and 15 micron, hence enabling specific filtration characteristics to be obtained as a function of the average diameter of the blood components to be removed by mechanical filtration. For example, the average leukocyte diameter is between 4 and 10 micron, the average erythrocyte diameter being between 7 and 8 as reported in and known from the literature.

The non-woven fabric layer coated with polyurethane can be sandwiched between hydrophobic layers of known type (or rather between layers of materials less hydrophilic than the non-woven fabric layer coated with polyurethane).

Because of the particular method of forming the filter, this can be obtained in a manner such as not to be dangerous and polluting to the environment.

This enables special costly control instruments for the impregnation process to be avoided, as can those disposal costs for the solvents or products used in the state of the art for the currently known impregnation of the filtering layer, for example of PBT. This cost saving is evidently reflected in the finished product costs.

The invention is further illustrated by the following examples of a filter according to the invention, given by way of non-limiting example.

From the said examples it was found that by using an aqueous dispersion of polyurethane instead of a solution in organic solvent, filters could be obtained having greater coating stability, demonstrated by the smaller values of the residue in water compared with similar values obtained from a solution in organic solvent.

Example 1

A dispersion of polyurethane of molecular weight between 12 and 18 kDa in water was used, with a polymer content of 5% by weight. This dispersion was obtained by the following process: the polyol and isocyanate components are dissolved in a ketonic solvent, then dispersed in water; the solvent is finally removed by distillation.

The polyurethane coating was applied to a non-woven fabric of PBT by immersion in the dispersion maintained at ambient temperature. Drying was carried out at 60°C. After 20 hours of drying, it was washed in osmotized water at 50°C. Subsequent drying was carried out at 60°C.

Example 2

A dispersion of polyurethane of molecular weight between 12 and 18 kDa in water was used, obtained by diluting at ambient temperature a polyurethane dispersion in water, with a PU content of 50% by weight and with the further addition of water to attain a polymer content of 0,5% by weight.

The polyurethane was NORETHANE WB 8301 produced by the firm NOVOTEX ITALIANA SPA.

The polyurethane coating was applied to a non-woven fabric of PBT by immersion in the dispersion maintained at ambient temperature. Drying was carried out at 50°C: After 20 hours of drying, it was washed in osmotized water at 45°C. Subsequent drying was carried out at 30°C.

Example 3

A dispersion of polyurethane of molecular weight between 12 and 18 kDa in water was used, with a polymer content of 5% by weight.

The polyurethane coating was applied to a non-woven fabric of PBT by immersion.

Drying was carried out at 100°C.

Example 4

Instead of a polyurethane dispersion in water, a solution of polyurethane of molecular weight between 12 and 18 kDa in isopropanol was used, with a polymer content of 3% by weight.

The polyurethane coating was applied to a non-woven fabric of PBT by immersion.

These examples are summarized in the following table:

| Example | PU Base | Polyurethane concentration |
|---|---|---|
| Example 1 | Water | 8% PU |
| Example 2 | Water | 0.5% PU |
| Example 3 | Water | 15% PU |
| Example 4 | Organic solvent | 3% PU |

**Effectiveness of leukocyte retention, platelet retention, and filtration times**

To compare filtration effectiveness, filters were produced suitable for large scale filtration of erythrocyte concentrates (laboratory filtration for blood banks). The filters were formed with a rigid plastic casing, each filter comprising 36 stacked layers each of 46 cm$^2$ surface, of the filtering material obtained in the stated examples. Bags of erythrocyte concentrates obtained by centrifugal separation from whole blood were filtered. The concentrates were used within the first 10 days from collection and were preserved at 4°C before filtration.

The results of the tests carried out were compared with those obtained with filters produced in accordance with EP189757 and WO2005113136, the data reported in these latter being utilized.

The leukocyte and platelet count were obtained by an automatic cell count before and after filtration. The post-filtration leukocyte count was determined by a count in a Nageotte chamber.

| Comparison of GVS filtrations with Fresenius patent (EP18975T1A1) and University of Southampton patent (WO2005113136A1) | | | |
|---|---|---|---|
| | GVS | Fresenius | Univ. **Southampton** |
| Patent | TBD | EP1897571A1 | WO2005113136A1 |
| Reference Example in Patent | Example 1 | Example 2 | Example 1 |
| Characteristics of the PU used | 15,000 Da < Mn < 17,500 | Mn < 10,000 Da | 20.000<Mw< 1,000,000 |
| Type of blood used for functional tests | Red Blood Cell Concentrates | Whole Blood | Whole blood |
| Size of samples used - small scale | Not available | Discs 3 cm in diameter 3 layers per filter 2 ml of blood | Discs 2 cm in diameter 9 layers per filter 4ml of whole blood filtered |
| Results | WBC removal % | 92% | 99.945% |
| | PLT removal % | 35% | 0% |

(continued)

| Comparison of GVS filtrations with Fresenius patent (EP18975T1A1) and University of Southampton patent (WO2005113136A1) | | | |
|---|---|---|---|
| | GVS | Fresenius | Univ. **Southampton** |
| Size of samples used - large scale | 36 layers surface 46 cm$^2$ | 40 layers surface 50 cm$^2$ | Not available |
| Results | Filtration time (min) | 17 | 14 |
| | PLT removal % | 95% | 100% |
| | Residual WBC/unit x 10$^6$ | 0.11 | 0.19 |

The comparison with the University of Southampton solution is possible only with small scale samples, also present in EP189757.

As can be seen from the table, the effectiveness of leukocyte (WBC) removal is much higher than that of EP189757.

**Evaluation of the residue in water**

The water soluble extracts were evaluated for each of the filters prepared in the above examples. With reference to the standard ISO 1135-4, Chapter 6, Chemical Requirements, three filters were connected in series with a peristaltic pump and water was recirculated for 2 hours at a flow rate of 1 l/h and a temperature of 37°C. Samples of permeated liquid were collected at the end of the test and sample absorbance was analyzed within the range 250-320 nm.

The results of the measurements are summarized in table 1. The WBC log reduction value was calculated by the formula:

$$\text{Log}_{10} \text{ (leukocyte number prefiltration/leukocyte number postfiltration).}$$

The platelet depletion value was calculated as:

$$\text{platelet depletion} = [(\text{platelet number postfiltration/µl}) \text{ / } (\text{platelet number prefiltration/µl})]\%$$

| Ex. | Treatment parameters | WBC Log reduction | Filtration n time | Platelet depletion | Absorb ance |
|---|---|---|---|---|---|
| Ex 1 | 8% PU in water base | 7.0 | 19.2 | 95% | <0.2 |
| Ex 2 | 0.5% PU in water base | 3.4 | 5.7 | 100% | <0.2 |
| Ex 3 | 15% PU in water base | 2.0 | 13.5 | 50% | <0.2 |
| Ex 4 | 3% PU in organic solvent base | 4.0 | 14.2 | 90% | >0.2 |

**Claims**

1. A filter for removing substances, in particular such as leukocytes and platelets, from blood or from blood derivatives, said filter comprising a casing containing a layered filter element comprising a plurality of layers, at least one layer of this latter being coated with polyurethane, said polyurethane having a molecular weight, **characterised in that** the polyurethane molecular weight is between 12,000 Dalton and 18,000 Dalton, preferably between 15,000 Dalton and 17,500 Dalton.

2. A filter as claimed in claim 1, **characterised in that** the polyurethane is obtained at least from an aliphatic isocyanate (isocyanate - N=C=O group) with characteristics similar to diphenylmethane diisocyanate (MDI); a polyol (typically a polyethylene glycol or polyesters) with which the isocyanate forms a urethane bond.

3. A filter as claimed in the preceding claims, **characterised in that** the polyurethane coated layer is of non-woven fabric such as polybutyleneterephthalate, polyesters or the like.

4. A filter as claimed in the preceding claims, **characterised in that** the polyurethane coated layer is sandwiched between less hydrophilic layers.

**Patentansprüche**

1. Filter zur Entfernung von Stoffen, insbesondere wie zum Beispiel Leukozyten und Blutplättchen, aus Blut oder Blutderivaten, wobei das genannte Filter ein Gehäuse umfasst, das ein, eine Vielzahl von Schichten unfassendes, geschichtetes Filterelement enthält, wobei mindestens eine Schicht davon mit Polyurethan bedeckt ist, wobei das genannte Polyurethan ein Molekulargewicht hat, **dadurch gekennzeichnet, dass** das Molekulargewicht des Polyurethans zwischen 12.000 und 18.000 Dalton, vorzugsweise zwischen 15.000 und 17.500 Dalton, liegt.

2. Filter nach Anspruch 1, **dadurch gekennzeichnet, dass** das Polyurethan aus mindestens einem aliphatischen Isocyanat (Isocyanatgruppe N=C=O) mit Merkmalen so ähnlich wie Diphenylmethan-diisocyanat (MDI); einem Polyol (üblicherweise einem Polyethylenglykol oder Polyester), mit dem das Isocyanat eine Urethanbindung bildet, erhalten ist.

3. Filter nach den vorhergehenden Ansprüchen, **dadurch gekennzeichnet, dass** die mit Polyurethan bedeckte Schicht aus Vliesstoff, wie zum Beispiel Polybutylenterephthalat, Polyester oder dergleichen ist.

4. Filter nach den vorhergehenden Ansprüchen, **dadurch gekennzeichnet, dass** die mit Polyurethan bedeckte Schicht zwischen weniger hydrophilen Schichten angeordnet ist.

**Revendications**

1. Filtre pour extraire des substances, en particulier telles que des leucocytes et des plaquettes, du sang ou de dérivés du sang, ledit filtre comprenant une enveloppe contenant un élément de filtrage en couches comprenant une pluralité de couches, au moins une couche de ce dernier étant revêtue de polyuréthane, ledit polyuréthane ayant un poids moléculaire, **caractérisé en ce que** le poids moléculaire du polyuréthane est compris entre 12.000 et 18.000 Dalton, préférablement entre 15.000 Dalton et 17.500 Dalton.

2. Filtre selon la revendication 1, **caractérisé en ce que** le polyuréthane est obtenu au moins d'un isocyanate aliphatique (groupe isocyanate N=C=O) avec des caractéristiques semblables au diisocyanate de diphénylméthane (MDI); un polyol (typiquement un polyéthylène glycol ou des polyesters) avec lequel l'isocyanate forme une liaison uréthane.

3. Filtre selon les revendications précédentes, **caractérisé en ce que** la couche revêtue de polyuréthane est un tissu non tissé tel que le polytéréphtalate de butylène, des polyesters ou similaire.

4. Filtre selon les revendications précédentes, **caractérisé en ce que** la couche revêtue de polyuréthane est intercalée entre des couches moins hydrophiles.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 4701267 A
- EP 329303 A
- EP 1897571 A
- WO 2005113136 A
- EP 189757 A
- EP 18975T1 A1
- WO 2005113136 A1
- EP 1897571 A1